# EUROPÄISCHE PATENTANMELDUNG

(11) **EP 0 777 117 A2**
(43) Veröffentlichungstag der Anmeldung: **04.06.1997**
(21) Anmeldenummer: 96250273.8
(22) Anmeldetag: 29.11.1996
(51) Int. Cl.: G01N 1/14, G01N 33/18

(54) **Vorrichtung zur Entnahme einer feststoffarmen Abwasserprobe**

(30) Priorität: 29.11.1995 DE 19546456; 22.02.1996 DE 19608295
(71) Anmelder: Dr. Bruno Lange GmbH, D-14163 Berlin (DE)
(72) Erfinder: Bittner, Klaus, 47802 Krefeld (DE); Kussmann, Michael, 40476 Düsseldorf (DE); Überbach, Otto, 40668 Meerbusch (DE)
(74) Vertreter: Christiansen, Henning, Dipl.-Ing.

(57) **Zusammenfassung**

Vorrichtung zur Entnahme einer feststoffarmen Abwasserprobe, insbesondere zur Messung der Phosphat-Konzentration im Abwasserbecken (1) einer Kläranlage, mit einem vibrationsarm gelagerten Sedimentationsgefäß (4) zur Aufnahme von Abwasser und zur Trennung von Schwebstoffen und Klarphase aus dem Abwasser mit einer Öffnung zum Einbringen des Abwassers, einer im oberen Bereich des Sedimentationsgefäßes (4) mündenden ersten Leitung (8) zur Entnahme einer Abwasserprobe aus der Klarphase, wobei das Sedimentationsgefäß (4) mindestens mit der Öffnung unterhalb des Flüssigkeitsspiegels im Abwasser angeordnet ist, die Öffnung in dem Sedimentationsgefäß (4), zur Beruhigung des aufgenommenen Abwassers während des Sedimentationszeitraums durch ein Ventil verschließbar ist oder einen Strömungswiderstand aufweist, der so groß ist, die Strömungsgeschwindigkeit im Sedimentationsgefäß (4) wesentlich kleiner ist als außerhalb.

## Beschreibung

Die Erfindung betrifft eine Vorrichtung zur Entnahme einer feststoffarmen Abwasserprobe, insbesondere zur Analyse gelöster Wasserinhaltsstoffe, gemäß dem Oberbegriff des Anspruchs 1.

In LARÖSCH, Jürgen; FRIES, Jörg: Phosphat-Meßanlage ohne Ultrafiltration; WLB - Wasser, Luft und Boden 12/1994 ist eine Meßanlage beschrieben, die die Messung der Phosphat-Konzentration im Abwasserbecken einer Kläranlage ermöglicht.

Hierbei wird aus einem Abwasserbecken der Kläranlage, vorzugsweise aus dem Denitrifikationsbecken, mittels einer Saugpumpe Abwasser in ein außerhalb des Abwasserbeckens angeordnetes Sedimentationsgefäß gepumpt. Das Sedimentationsgefäß wird dann während eines Sedimentationszeitsraums vibrationsarm gelagert, so daß sich die im Abwasser suspendierten Schwebstoffe aufgrund der Schwerkraft am Boden des Sedimentationsgefäßes absetzen und im oberen Bereich des Sedimentationsgefäßes eine schwebstoffarme sogenannte Klarphase entsteht. Die vibrationsarme Lagerung des Sedimentationsgefäßes während der Sedimentationszeit ist hierbei wichtig, da andernfalls im Sedimentationsgefäß Strömungen entstehen, die ein Absetzen der Schwebstoffe verhindern oder zumindest zeitlich verzögern.

Mittels einer zweiten Pumpe wird dann aus der Klarphase eine schwebstoffarme Abwasserprobe entnommen, die anschließend photometrisch auf ihre Phosphat-Konzentration hin untersucht wird. Hierzu wird der Abwasserprobe eine schwefelsaure Molybdat-Vanadium-Lösung zugegeben, die mit dem Abwasser zu der gelben Phosphor-Vanadomolybdänsäure (P-V-Mo-Säure) reagiert, wobei die Intensität der gelben Einfärbung und damit der Lichtabsorption von der Phosphat-Konzentration im Abwasser abhängt. Anschließend wird die Abwasserprobe durchleuchtet, wobei mittels einer Photozelle die Lichtabsorption der Abwasserprobe gemessen und daraus die Phosphat-Konzentration berechnet wird.

Für die Durchführung des photometrischen Meßverfahrens ist die vorherige Abscheidung der Schwebstoffe wichtig, da andernfalls die gemessene Lichtabsorption und damit die ermittelte Phosphat-Konzentration durch die Lichtabsorption bzw. -streuung an den Schwebstoffen verfälscht wird.

Das vorbekannte Verfahren ermöglicht vorteilhaft eine automatische und regelmäßige Messung der Phosphat-Konzentration im Abwasser eines Klärwerks und damit zum einen eine laufende Überwachung der Abwasserqualität und zum anderen eine exakte Dosierung der Phosphat-Fällmittel zur Senkung der Phosphat-Konzentration im Abwasser. Dies ist wichtig, da die Phosphat-Fällmittel in der Regel umweltschädlich sind und somit einerseits eine Überdosierung der Phosphat-Fällmitel verhindert werden soll, andererseits aber die Dosierung der Phosphat-Fällmittel hinreichend hoch sein muß, um die Phosphat-Konzentration unter den zulässigen Wert zu senken.

Das vorbekannte Verfahren weist jedoch verschiedene Nachteile auf:

Bei jeder Messung muß das Sedimentationsgefäß mit Abwasser gefüllt werden. Dies geschieht - wie oben beschrieben - indem eine Pumpe über eine Leitung Abwasser aus dem Abwasserbecken in das Sedimentationsgefäß pumpt. Da jedoch die Leitung in der Regel noch mit dem Abwasser der letzten Messung gefüllt ist, muß vor der Füllung des Sedimentationsgefäßes zunächst die gesamte Leitung vollständig mit neuem Abwasser gefüllt werden, damit die neue Messung nicht durch das alte Abwasser verfälscht wird. Das von den Pumpen bei jeder Messung zu bewältigende Pumpvolumen besteht also nicht nur aus dem Füllungsvolumen des Sedimentationsgefäßes, sondern auch aus dem Volumen der Leitung. Zum einen macht dies eine relativ starke Förderpumpe mit entsprechend großem Energieverbrauch erforderlich, zum anderen ist durch das relativ große Pumpvolumen eine Vorlaufzeit vor jeder Messung nötig und damit die maximale Meßrate nach oben begrenzt.

Ein weiterer Nachteil ist darin zu sehen, daß bei einer Probenentnahme aus Abwasser mit schlammartiger Konsistenz durch die relativ starke Förderpumpe und die zur vollständigen Neufüllung der Förderleitung vor jeder Messung erforderliche Förderung relativ großer Abwassermengen die Schlammstruktur negativ beeinflußt wird. So werden die im Abwasser normalerweise relativ großen Schlammflocken durch die beim Pumpen auftretenden Turbulenzen verkleinert, was die anschließende Sedimentation verzögert.

Der Erfindung liegt deshalb die Aufgabe zugrunde, eine Vorrichtung zu schaffen, die eine rasche Behandlung einer schwebstoffarmen Abwasserprobe im Rahmen der Probenentnahme ermöglicht.

Die Aufgabe wird durch die kennzeichnenden Merkmale des Anspruchs 1 gelöst.

Die Erfindung schließt die technische Lehre ein, ein Sedimentationsgefäß im Abwasser gegen Verwirbelungen und Einströmungen geschützt anzuordnen und mittels einer im oberen Bereich des Sedimentationsgefäßes mündenden Leitung eine Abwasserprobe aus der Klarphase zu entnehmen.

Die erfindungsgemäße Vorrichtung sieht ein Sedimentationsgefäß vor, das im Abwasser - beispielsweise im Abwasserbecken einer Kläranlage - angeordnet ist und eine Öffnung zum Auffüllen mit Abwasser aufweist, wobei das Sedimentationsgefäß derart angeordnet ist, daß sich zumindest diese Auffüllöffnung unterhalb des Wasserspiegels befindet.

Wichtig ist hierbei, daß das Sedimentationsgefäß vibrationsarm gelagert ist, damit sich die in dem Abwasser enthaltenen Schwebstoffe während des Sedimentationszeitraums am Boden des Sedimentationsgefäßes absetzen können, so daß sich im oberen Bereich des Sedimentationsgefäßes eine feststoffarme Klarphase bildet.

Bei der Erfindung ist besonders vorteilhaft, daß die zu sedimentierenden Schwebstoffe nicht auf einem langen Transportweg in engen Rohrleitungen oder Pumpen zerbrochen werden, wodurch eine spätere Trennung beeinträchtigt ist. Durch den Erhalt der vollen Größe der Schwebstoffteilchen ist der Sedimentationsvorgang auch beschleunigt.

Damit der Sedimentationsprozeß durch die Abwasserströmungen außerhalb des Sedimentationsgefäßes nicht behindert wird ist die Öffnung während des Sedimentationszeitraums durch ein Ventil verschließbar oder weist einen Strömungswiderstand auf, der so groß ist, daß die Strömungsgeschwindigkeit außerhalb des Sedimentationsgefäßes wesentlich größer als innerhalb des Sedimentationsgefäßes.

Zur Entnahme einer Abwasserprobe aus der Klarphase weist die erfindungsgemäße Vorrichtung eine erste Leitung auf, die im oberen Bereich des Sedimentationsgefäßes mündet.

In einer Variante der Erfindung ist diese Leitung mit einer außerhalb des Abwasserbeckens angeordneten Saugpumpe verbunden, die die Abwasserprobe aus der während des Sedimentationszeitraums im Sedimentationsgefäß sich bildenden Klarphase absaugt. Die Förderhöhe weist hierbei eine theoretisch maximal erreichbare Obergrenze von ca. 10 m auf.

Um auch eine größere Förderhöhe erreichen zu können, ist deshalb in einer anderen Variante eine zweite Leitung vorgesehen, die ebenfalls in dem Sedimentationsgefäß mündet.

Zur Entnahme einer Abwasserprobe wird hierbei zunächst die Öffnung des Sedimentationsgefäßes durch ein Ventil verschlossen und anschließend Luft über die zweite Leitung in das Sedimentationsgefäß gepumpt. Durch den dabei im Sedimentationsgefäß entstehenden Überdruck wird die Abwasserprobe in die erste Leitung hineingepreßt.

In einer bevorzugten Ausführungsform dient die zweite Leitung zusätzlich zur Befüllung des Sedimentationsgefäßes vor Beginn des Sedimentationsprozeßes. Hierzu wird durch eine mit der zweiten Leitung verbundenen Saugpumpe Luft aus dem Sedimentationsgefäß abgesaugt, so daß in dem Sedimentationsgefäß ein Unterdruck entsteht und Abwasser in das Sedimentationsgefäß eintritt.

Die erfindungsgemäße Vorrichtung bietet die Möglichkeit, automatisch in regelmäßigen - und verhältnismäßig kurzen - Zeitabständen Abwasserproben zu entnehmen. Damit die Messung nicht durch das noch von der letzten Messung im Sedimentationsgefäß verbliebene Abwasser verfälscht wird, muß jeweils das Abwasser der letzten Messung aus dem Sedimentationsgefäß entfernt und neues Abwasser eingefüllt werden.

Eine Variante der Erfindung sieht deshalb vor, daß Sedimentationsgefäß vor jeder Messung mit einer Spülflüssigkeit - vorzugsweise Leitungswasser - zu spülen.

Bei dem vorbekannten Verfahren ist das Sedimentationsgefäß außerhalb des Abwassers angeordnet und wird über eine Förderleitung vor jedem Sedimentationsvorgangs mit Abwasser gefüllt. Da das Abwasser eine hohe Konzentration an Schwebstoffen und in der Praxis teilweise eine schlammartige Konsistenz aufweist, sind die zu messenden Eigenschaften innerhalb des Abwassers nicht homogen, sondern variieren räumlich. Aus diesem Grund ist die Förderung einer relativ großen Menge Abwassers über einen großen Ansaugquerschnitt erforderlich, um die räumliche Variation der zu messenden Größe innerhalb des Abwassers auszugleichen und das Sedimentationsgefäß mit Abwasser zu füllen, das einen repräsentativen räumlichen Mittelwert darstellt. Der Querschnitt und damit das Volumen der Förderleitung ist deshalb bei der vorbekannten Vorrichtung relativ groß.

Die Anwendung der erfindungsgemäßen Idee der Spülung einer Leitung würde deshalb bei der vorbekannten Vorrichtung ein relativ großes Volumen an Spülflüssigkeit erfordern.

Bei der erfindungsgemäßen Vorrichtung ist hingegen lediglich die Probenentnahmeleitung zu spülen, über die mittels der Dosierpumpe Abwasser aus der Klarphase des Sedimentationsgefäßes entnommen wird. Da in der Klarphase die Konzentration an Schwebstoffen jedoch sehr gering ist, ist die Klarphase entsprechend homogen mit räumlich kaum variierenden Eigenschaften. Aus diesem Grund kann die Probenentnahme an einem räumlich scharf abgegrenzten Punkt innerhalb der Klarphase erfolgen und damit die Probenentnahmeleitung einen sehr geringen Querschnitt aufweisen mit der Folge eines geringen zu spülenden Volumens. Die Probenentnahmeleitung kann also bei der erfindungsgemäßen Vorrichtung vorteilhaft relativ schnell und mit geringem Aufwand gespült werden.

In einer Variante der Erfindung ist die Auffüllöffnung zur Beruhigung des im Sedimentationsgefäß befindlichen Abwassers zumindest während des Sedimentationszeitraums so ausgeführt, daß deren Strömungswiderstand so groß ist, daß die Strömungsgeschwindigkeit innerhalb des Sedimentationsgefäßes wesentlich geringer ist als außerhalb. Dies ist erforderlich, damit außerhalb des Sedimentationsgefäßes auftretende Strömungen, wie sie beispielsweise in Abwasserbecken von Klärwerken auftreten, den Sedimentationsprozeß nicht in einem Maße behindern, das die erfindungsgemäße Vorrichtung funktionsunfähig macht.

Eine andere Ausführungsform sieht ein federbelastetes Ventil vor, das die Auffüllöffnung im Normalzustand verschließt und erst beim Überschreiten einer bestimmten Druckdifferenz zwischen Innenseite und Außenseite des Sedimentationsgefäßes öffnet. Die Federkraft ist dabei so bemessen, daß die aufgrund von Strömungen im Abwasser auftretenden Druckdifferenzen nicht ausreichen, um das Ventil zu öffnen. Das Ventil öffnet erst dann, wenn die Förderpumpe arbeitet, um entweder Abwasser in das Sedimentationsgefäß einzusaugen oder von der letzten Messung noch im Sedimentationsgefäß verbliebenes Abwasser oder Spülwasser auszupumpen. Um das Ventil zu öffnen ist deshalb eine bestimmte minimale Pumpleistung erforderlich, die auch während des Pumpvorgangs im geöffneten Zustand des Ventils nicht unterschritten werden darf, damit das Ventil nicht wieder schließt. Die Pumpe muß also gegen die Federkraft anarbeiten, die das Bestreben hat, das Ventil wieder zu schließen.

In einer Ausführungsform der Erfindung ist deshalb ein Ventil vorgesehen, das - wie bereits das vorstehend beschriebene Ventil - aufgrund einer Federkraft im Normalzustand geschlossen ist und sich erst beim Überschreiten einer bestimmten Druckdifferenz zwischen Innen- und Außenseite des Sedimentationsgefäßes öffnet. Der Unterschied zu dem vorstehend beschriebenen Ventil besteht hierbei darin, daß die Rückstellkraft, die das Bestreben hat, das Ventil wieder zu schließen, im geöffneten Zustand des Ventils relativ gering ist. Beim Öffnen des Ventil muß also lediglich ein "Totpunkt" überschritten werden, ab dem dann die Rückstellkraft relativ gering ist und lediglich ausreicht, um das Ventil bei den zwischen Innen- und Außenseite des Sedimentationsgefäßes im Normalbetrieb auftretenden Druckdifferenzen wieder zu schließen. Bei dieser Ausführungsform muß die zum Öffnen des Ventils erforderliche relativ hohe Pumpleistung also nur kurzzeitig aufgebracht werden.

In einer weiteren Variante der Erfindung ist vorgesehen, die Auffüllöffnung in dem Sedimentationsgefäß im wesentlichen quer zur Hauptströmungsrichtung anzuordnen. So verläuft die Hauptströmungsrichtung in Abwasserbecken von Kläranlagen in der Regel horizontal. Dies liegt daran, daß zum einen das Abwasserbecken in horizontaler Richtung eine wesentlich größere Erstreckung aufweist als in vertikaler Richtung und zum anderen das Abwasser in dem Abwasserbecken durch einen rotierenden Rechen umgerührt wird. Die Auffüllöffnung wird deshalb vorzugweise an der Unterseite des Sedimentationsgefäßes mit horizontal verlaufender Querschnittsfläche angeordnet. Hierdurch wird einem Eintreten der Strömung in das Sedimentationsgefäß und damit einer Störung des Sedimentationsvorgangs entgegengewirkt.

Sowohl bei der Förderleitung als auch bei der Probenentnahmeleitung kann das Abwasser bei Außentemperaturen unterhalb des Gefrierpunkts in der Leitung einfrieren. Zum einen wird hierdurch die Messung verhindert, zum anderen besteht die Gefahr, daß die Leitungen wegen des sich beim Einfrieren ausdehnenden Abwassers gesprengt werden.

Eine bevorzugte Ausführungsform der Erfindung sieht deshalb eine Heizeinrichtung für die Probenentnahmeleitung und/oder die Förderleitung vor. Diese kann beispielsweise aus einer die Leitungen zumindest auf einem Teil ihrer Länge umhüllenden elektrisch betreibbaren Heizmanschette bestehen. Hierdurch wird vorteilhaft ein automatischer Betrieb der erfindungsgemäßen Vorrichtung beispielsweise in einem Klärwerk zur laufenden Entnahme und Analyse von Wasserproben auch bei Außentemperaturen unterhalb des Gefrierpunkts ermöglicht.

In einer bevorzugten Ausführungsform dieser Variante sind die elektrischen Heizdrähte aus einem Kaltleiter gefertigt und weisen somit einen mit steigender Temperatur zunehmenden elektrischen Widerstand auf. Hierdurch wird die Temperatur der beheizten Leitung vorteilhaft stabilisiert. Bei einer geringen Temperatur ist der elektrische Widerstand der Heizdrähte entsprechend gering und damit die in den Heizdrähten in Wärme umgesetzte elektrische Leistung entsprechend groß. Mit zunehmender Temperatur der beheizten Leitung und damit auch der Heizdrähte nimmt jedoch der elektrische Widerstand der Heizdrähte zu und damit die Heizleistung ab, so daß die Temperatur der Leitung auf einem Nennwert stabilisiert wird.

In einer Variante der Erfindung von eigener schutzwürdiger Bedeutung ist das Sedimentationsgefäß als im wesentlichen rotationssymmetrischer Hohlkörper ausgeführt, der um seine Symmetrieachse drehbar gelagert ist. Weiterhin ist in dieser Variante ein Antrieb vorgesehen, der das Sedimentationsgefäß während des Sedimentationszeitraums um seine Symmetrieachse in Drehung versetzt. Dadurch wird auch das im Sedimenationsgefäß befindliche Abwasser in Drehung versetzt. Auf das Abwasser wirkt also zusätzlich zur Gravitationskraft auch die radial nach außen gerichtete Zentrifugalkraft, so daß sich die Schwebstoffe bei hinreichend großer Drehgeschwindigkeit am Umfang des Sedimentationsgefäßes ablagern, während die Klarphase achsnah gelegen ist. Die Probenentnahmeleitung ist deshalb mit ihrer Mündungsöffnung ebenfalls achsnah, vorzugsweise auf der Symmetrieachse des Sedimentationsgefäßes angeordnet. Die Entnahme der Abwasserprobe kann hierbei wahlweise bei rotierendem Sedimentationsgefäß oder nach dem Anhalten der Drehbewegung im Ruhezustand des Sedimentationsgefäßes erfolgen. Die Entnahme der Abwasserprobe aus dem rotierenden Sedimentationsgefäß bietet dabei den Vorteil, daß kein Sediment von der Wand des Sedimentationsgefäßes in achsnahe Bereiche zurückfallen und die Messung verfälschen kann.

Bei dieser Variante der Erfindung wird das im Sedimentationsgefäß befindliche Abwasser während des Sedimentationszeitraums in Drehung versetzt. In einer bevorzugten Ausführungsform dieser Variante ist deshalb vorgesehen, an der Innenwand des Sedimentationsgefäßes Mitnehmer anzuordnen, die das Abwasser bei einer Drehung des Sedimentationsgefäßes "mitnehmen" und ebenfalls in Drehung versetzen. Diese Mitnehmer sind vorzugsweise als schaufelartige Platten ausgeführt, die an der Innenwand des Sedimentationsgefäßes angeordnet sind und sich in axialer Richtung zumindest über einen Teil der Länge des Sedimentationsgefäßes erstrecken. In radialer Richtung erstrecken sich die Mitnehmer von der Wand des Sedimentationsgefäßes so weit nach innen, daß in der Mitte des Sedimentationsgefäßes nahe der Rotationsachse ein Bereich frei bleibt, in dem mittels der Probenentnahmeleitung eine Probe aus der Klarphase entnommen wird.

In einer bevorzugten Ausführungsform ist vorgesehen, die Funktionen der drehbaren Lagerung des Sedimentationsgefäßes und der Förderleitung in einem Bauteil zu vereinen. Hierzu ist die Förderleitung als hohlzylindrische Welle ausgebildet, die so an dem rotationssymmetrischen Sedimentationsgefäß angeschlossen ist, daß die Symmetrieachse des Sedimentationsgefäßes und die Längsachse der Hohlwelle im wesentlichen fluchten. Die Hohlwelle ist drehbar gelagert und durch einen Motor antreibbar. Bei einer Drehung des Sedimentationsgefäßes auf der Hohlwelle wirkt nun auf die im Abwasser suspendierten Schwebstoffe die radial nach außen gerichtete Zentrifugalkraft, so daß sich die Schwebstoffe an der Außenwand des Sedimentationsgefäßes ablagern. Die Klarphase ist dagegen achsnah gelegen, so daß durch die Hohlwelle eine schwebstoffarme Abwasserprobe abgesaugt werden kann.

In den vorstehend beschriebenen Varianten der Erfindung ist die aus dem Sedimentationsgefäß entnommene Abwasserprobe relativ feststoffarm, was Voraussetzung für eine Durchführung photometrischer Untersuchungsverfahren ist, da die durch die Feststoffanteile verursachte Trübung andernfalls die Meßergebnisse verfälschen würde. Insbesondere bei der Entnahme der Abwasserprobe aus einem stark schlammhaltigen Gewässer kann es jedoch vorkommen, daß der Feststoffgehalt der Abwasserprobe zwar gegenüber dem Feststoffgehalt des Entnahmegewässers stark verringert ist, aber trotzdem für die Durchführung einer photometrischen Untersuchung zu hoch ist. In einer vorteilhaften Variante der Erfindung wird die aus der Klarphase des Sedimentationsgefäßes entnommene Abwasserprobe deshalb zunächst in einem Auffanggefäß gesammelt, in dem der Feststoffgehalt der Abwasserprobe gemessen wird, um beurteilen zu können, ob die Qualität der Abwasserprobe für die nachfolgenden Untersuchungsverfahren ausreicht. Die Bestimmung des Feststoffgehalts erfolgt vorzugsweise durch Messung der Trübung der Abwasserprobe mittels eines Photometers. Überschreitet die von dem Photometer gemessene Trübung der Abwasserprobe einen vorgegebenen Schwellwert, so reicht die Qualität der Abwasserprobe für die nachfolgenden Untersuchungsverfahren nicht aus. In diesem Fall ist es zur Verbesserung der Probenqualität beispielsweise möglich, der Abwasserprobe über einen Dispenser ein Fällungsmittel zuzusetzen, das dafür sorgt, daß sich die restlichen Feststoffanteile der Abwasserprobe nach kurzer Zeit am Boden des Auffanggefäßes absetzen. Da das Fällungsmittel hierbei lediglich dem relativ kleinen Flüssigkeitsvolumen des Auffanggefäßes zugesetzt wird, nicht jedoch dem gesamten Entnahmegewässer, ist vorteilhaft nur eine geringe Menge des Fällungsmittels erforderlich.

Eine zusätzliche Ausführungsform der Erfindung schließt die Erkenntnis ein, daß sich bei einem Sedimentationsvorgang in Abwasser bei einer Vergrößerung der für eine Sedimentation notwendigen, sich waagerecht erstreckenden Wandungsfläche des verwendeten Sedimentationsgefäßes bei gleichem Abwasservolumen die sich über dem Sediment bildende Klarphase zwar in ihrem Volumen, jedoch nicht in ihrem Höhenmaß vergrößert. Gleichermaßen führt eine Vergrößerung der vorstehend genannten Wandung des Sedimentationsgefäßes zu keiner Verkürzung der Sedimentationszeit.

Es wurde jedoch in überraschender Weise gefunden, daß eine Erweiterung in Richtung auf den Gefäßboden im Sedimentationsbereich in Form eines umgekehrten Trichters zu einer erheblichen Beschleunigung des Sedimentationsprozesses führt. Dies ist deswegen erstaunlich, da die meisten Arten physikalischer Einwirkung zu einer Verlangsamung der Sedimentation führen. Dadurch daß den sich im unteren Bereich konzentrierenden Schwebstoffen sich ein erweitertes Volumen zur Verfügung steht, läßt sich deren Absinkgeschwindigkeit beträchtlich vergrößern. Damit läßt sich eine fast vollständige Sedimentation bereits zu Zeiten erreichen, bei denen unter anderen Umständen sich erst ein Bruchteil der Schwebteilchen abgesetzt haben würde.

Die geneigte Gefäßwandung im Sedimentationsbereich stellt eine Vergrößerung der Oberfläche dar. Auch an dieser Oberfläche bildet sich nach Absinken der im Abwasser befindlichen Schlamm- und/oder Feststoffe eine Klarphase. Aufgrund der Dichteunterschiede zwischen Wasser und Schlamm- und/oder Feststoffen steigt das im wesentlichen schlamm- und/oder feststofffreie Wasser an der geneigten Gefäßwandung in dem Sedimentationsgefäß nach oben. Gleichzeitig drängt dieses Wasser dabei Schlamm- und/oder Feststoffpartikel aus höherliegenden Schichten des in dem Sedimentationsgefäß befindlichen Flüssigkeit zur Sedimentation.

Je stärker sich die Schlamm- und/oder Feststoffpartikel verdichten, je langsamer verläuft der weitere Sedimentationsvorgang. Betrachtet man die Höhe des durch die Schlamm- und/oder Feststoffpartikel bestimmten Pegels bei gleicher Verdichtung bei einem herkömmlichen mit dem bei einem erfindungsgemäßen Sedimentationsgefäß, so liegt der Pegel der sedimentierten Schlamm- und/oder Feststoffpartikel bei dem gemäß der Erfindung kegelstumpfförmig ausgebildeten Sedimentationsgefäß tiefer. D.h. der Schlamm verdichtet sich in einem in Richtung des Gefäßbodens erweiternden Sedimentationsgefäß bezogen auf den gleichen Zeitraum in einem geringeren Maß und sinkt daher noch schneller ab.

Durch die gegeneinander und gegenüber der den Gefäßboden bildenden und zur Sedimentation zur Verfügung stehenden waagerechten Wandungsfläche geneigten Wandungsteile des Sedimentationsgefäßes, ist gleichzeitig ein Anheben des Höhenniveaus der sich nach erfolgter Sedimentation aus dem Abwasservolumen bildenden Klarphase erreichbar. Eine höherer Wasserstand der Klarphase erleichtert in günstiger Weise die Entnahme einer schlamm- und/oder schwebstoffreien Probe zu Meßzwecken.

Der besondere Vorteil der vorstehend erläuterten Ausführungsform der Erfindung besteht darin, daß auch bei relativ schlecht sedimentierenden Schlämmen einerseits eine Reduzierung der Sedimentationszeit auf nahezu die Hälfte der Zeit erreichbar ist, welche bis zum Abschluß der Sedimentation in einem zylindrischen Sedimentationsgefäß der herkömmlichen Art erforderlich wäre, und andererseits gleichzeitig ein für eine bequeme Entnahme ausreichendes Höhenniveau des Klarphasenvolumens vorhanden ist. Mit dieser Maßnahme ist eine Taktzeit im Rahmen der Sedimentation von ca. 10 min in fast allen Fällen erreichbar. Für die Sedimentation steht dabei ein Zeitanteil von ca. 80% zur Verfügung.

Entsprechend dieser Ausführungsform der Erfindung weist das Sedimentationsgefäß einen geschlossenen Gefäßboden und eine im wesentlichen entsprechend einem umgekehrten Trichter ausgebildete Zone auf, welche sich, vorzugsweise in Form eines Kegelstumpfes in Richtung des Gefäßbodens erweitert. An die kegelstumpfförmigen Zone schließt sich eine zylindrische, den Gefäßboden tragende Zone geringer Länge an.

Nach einer günstigen Weiterbildung der Erfindung stimmt die Grundfläche der kegelstumpfförmigen Zone mit dem Boden des Sedimentationsgefäßes überein.

Das Sedimentationsgefäß weist erfindungsgemäß eine erste und eine zweite Leitung einerseits zwecks Entnahme einer Probe aus der Klarphase und andererseits zwecks Verbindung mit einer einen Unterdruck zum Einbringen eines die Klarphase liefernden Flüssigkeit in das Sedimentationsgefäß oder einen Überdruck zum Herausfördern dieses Flüssigkeit aus dem Sedimentationsgefäß erzeugenden Einrichtung, beispielsweise mit einer in der Drehrichtung steuerbaren Förderpumpe, auf.

Die erste und die zweite Leitung sind - sich in Richtung der Längsachse des Sedimentationsgefäßes erstreckend - in der oberen, dem Gefäßboden gegenüberliegenden, Zone angeordnet, wobei die erste Leitung auf gleicher Achse liegend innerhalb der zweiten Leitung positioniert ist und eine im wesentlichen axiale Probenentnahme aus dem Sedimentationsgefäß ermöglicht. Die innenliegende Leitung ist innerhalb der Rohr-in-Rohr-Anordnung durch Abstandhalter fixiert.

Entsprechend einer vorteilhaften Weiterbildung der Erfindung ist die erste Leitung zur Entnahme einer Probe aus der Klarphase in der Wandung der kegelstumpfförmigen Zone angeordnet, so daß die die Entnahme in im wesentlichen radialer Richtung erfolgt. Bauliche Vorteile ergeben sich hierbei insbesondere dadurch, daß die konstruktiv relativ aufwendige Rohr-in-Rohr-Führung der beiden vorgesehenen Leitungsanschlüsse entfällt.

Für das Ein- und Ausbringen des zu kontrollierenden Flüssigkeit in das Sedimentationsgefäß ist entsprechend der bevorzugten Ausführungsform der Erfindung im Gefäßboden eine, beispielsweise durch ein Magnetventil, steuerbare Verschlußeinrichtung vorgesehen. Die zeitliche Koordinierung des Betriebs der die entsprechenden Druckbedingungen für Ein- und Ausbringen der Abwassermenge in das Sedimentationsgefäß erzeugenden Pumpeneinrichtung und der Aktivierung des Magnetventils der Verschlußeinrichtung am Gehäuseboden erfolgt über eine gesonderte Steuerung.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als Ausführungsbeispiel der Erfindung eine Probenentnahmevorrichtung im Abwasserbecken eines Klärwerks in perspektivischer Darstellung,
- Figur 2: das Ausführungsbeispiel aus Figur 1 im Querschnitt,
- Figur 3: ein Sedimentationsgefäß als Teil eines Ausführungsbeispiels der Erfindung in perspektivischer Darstellung,
- Figur 4: eine andere Ausführungsform eines erfindungsgemäßen Sedimentationsgefäßes.
- Figur 5a: ein weiteres Ausführungsbeispiel eines erfindungsgemäßen Sedimentationsgefäßes in der Seitenansicht,
- Figur 5b: einen Teil des Sedimentationsgefäßes aus Figur 5 detailliert in Querschnittsdarstellung.
- Figur 6: eine zusätzliche Ausführungsform des erfindungsgemäßen Sedimentationsgefäßes in Schnitt,
- Figur 7: eine vorteilhafte Weiterbildung des in Figur 6 gezeigten Sedimentationsgefäßes als Teilschnitt,
- Figur 8: eine weitere Variante des erfindungsgemäßen Sedimentationsgefäßes in perspektivischer Darstellung, sowie
- Figur 9: als Ausführungsbeispiel die Teilschnittdarstellung einer Kläranlage mit Abwasserbecken und einer Probenentnahmeeinrichtung gemäß Figur 7.

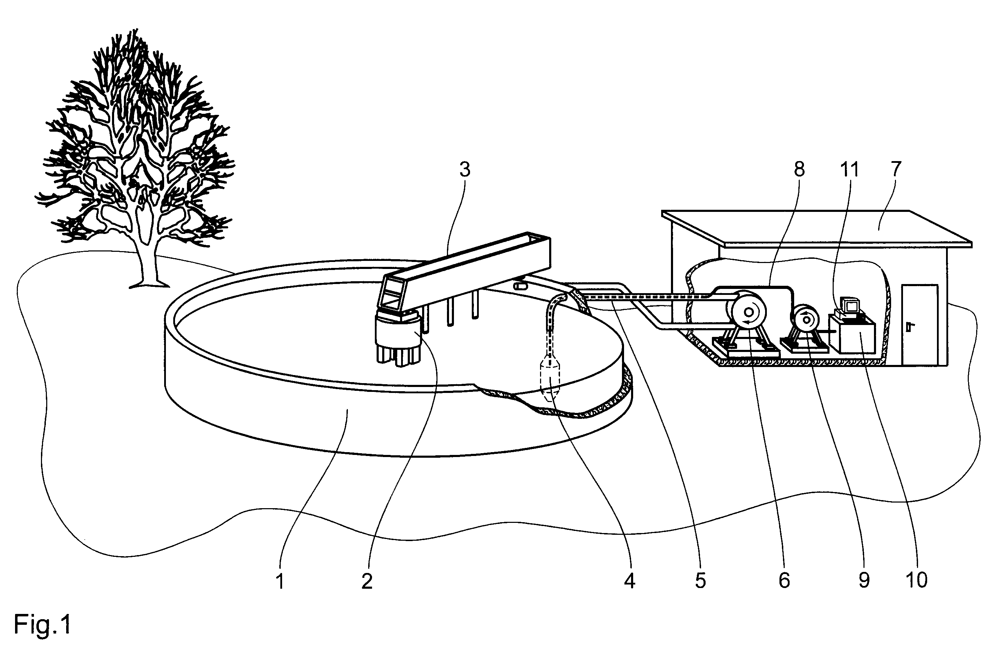

In dem in Figur 1 dargestellten Ausführungsbeispiel dient die erfindungsgemäße Vorrichtung zur Entnahme einer schwebstoffarmen Abwasserprobe zur Messung der Phosphat-Konzentration im Abwasserbecken 1 eines Klärwerks.

Das Abwasserbecken 1 ist im wesentlichen zylindrisch ausgeführt und besteht aus Beton. Der Durchmesser des Abwasserbeckens 1 beträgt ca. 20 m bei einer Höhe von ca. 2 m, wobei das Abwasserbecken 1 ca. 1 m tief ins Erdreich eingelassen ist.

In der Mitte des Abwasserbeckens 1 ist eine ebenfalls aus Beton bestehende Säule 2 angeordnet, auf deren Oberseite ein das Abwasserbecken 1 in radialer Richtung überspannender Ausleger 3 um die Hochachse drehbar gelagert ist. Dieser Ausleger 3 ist an seiner Außenseite mittels zweier Zahnräder auf einer Zahnschiene gelagert, die auf der Oberseite der Außenwand des Abwasserbeckens umläuft. Die beiden Zahnräder werden durch einen Elektromotor angetrieben und ermöglichen dadurch, den Ausleger 3 um die Hochachse zu drehen. An der Unterseite des Auslegers 3 sind mehrere nach unten in das Abwasser hineinragende Rechen angeordnet, die bei einer Drehung des Auslegers 3 das im Abwasserbecken 1 befindliche Abwasser umrühren.

Am Rand des Abwasserbeckens 1 ist unterhalb des Wasserspiegels ein Sedimentationsgefäß 4 angeordnet, das als rotationssymmetrischer Hohlkörper ausgeführt ist, wobei die Hochachse des Sedimentationsgefäßes 4 im wesentlichen senkrecht verläuft. Entlang der Hochachse verjüngt sich das Sedimentationsgefäß 4 und läuft nach oben in der Ansaugöffnung und nach unten in der Auffüllöffnung aus.

Die Ansaugöffnung ist mit einer Förderleitung 5 verbunden, die durch die Wandung des Abwasserbeckens 1 hindurchgeführt und an ihrem anderen Mündungsende an eine Förderpumpe 6 angeschlossen ist, die in einem neben dem Abwasserbecken 1 gelegenen Maschinenhaus 7 untergebracht ist. Diese Förderpumpe 6 arbeitet beim Befüllen des Sedimentationsgefäßes 4 als Saugpumpe und erzeugt im Sedimentationsgefäß 4 einen Unterdruck, der Abwasser von unten durch die Auffüllöffnung in das Sedimentationsgefäß 4 eintreten läßt.

Nach dem Befüllen des Sedimentationsgefäßes 4 wird die Förderpumpe 6 abgestellt, damit sich das im Sedimentationsgefäß 4 befindliche Abwasser beruhigen kann. Im Abwasserbecken 1 herrschen in der Regel relativ starke Strömungen, die die Beruhigung des im Sedimentationsgefäß 4 befindlichen Abwassers und damit den Sedimentationsvorgang stören können. Dem wird dadurch entgegengewirkt, daß das Sedimentationsgefäß 4 bis auf die Einfüllöffnung geschlossen ist und wegen des relativ kleinen Querschnitts der Einfüllöffnung einer von außen eindringenden Strömung einen großen Strömungswiderstand entgegensetzt.

Darüber hinaus ist die Auffüllöffnung an der Unterseite des Sedimentationsgefäßes 4 mit horizontal verlaufender Querschnittsfläche angeordnet. Die Hauptströmungsrichtung im Abwasserbecken 1 verläuft jedoch in horizontaler Richtung. Die Ursache hierfür ist zum einen darin zu sehen, daß die räumliche Erstreckung des Abwasserbeckens 1 in horizontaler Richtung wesentlich größer ist als in senkrechter Richtung. Zum anderen wird das Abwasser durch den an dem Ausleger 3 angebrachten Rechen umgerührt. Da die Hauptströmungsrichtung somit rechtwinklig zu der Einflußrichtung an der Auffüllöffnung verläuft, wird einer Störung des im Sedimentationsgefäß 4 befindlichen Abwassers während des Sedimentationsvorgangs entgegengewirkt.

Aufgrund der Schwerkraft lagern sich dann die im Abwasser suspendierten Schwebstoffe am Boden des Sedimentationsgefäßes 4 ab oder fallen durch die Auffüllöffnung nach unten aus dem Sedimentationsgefäß 4 aus. Die Konzentration an Schwebstoffen innerhalb des Sedimentationsgefäßes 4 nimmt deshalb von unten nach oben ab. Nach dem Ende des Sedimentationszeitraums liegen schließlich im Sedimentationsgefäß 4 zwei räumlich relativ scharf abgegrenzte Phasen vor. Im oberen Bereich des Sedimentationsgefäßes 4, der sogenannten Klarphase, ist die Konzentration an Schwebstoffen sehr gering. Im unteren Bereich des Sedimentationsgefäßes 4, insbesondere am Boden des Sedimentationsgefäßes 4, hat sich dagegen eine Sedimentschicht abgelagert.

Im Inneren der Förderleitung 5 verläuft im wesentlichen koaxial zu dieser eine Probenentnahmeleitung 8, die zur Entnahme einer Abwasserprobe aus der Klarphase des im Sedimentationsgefäß 4 befindlichen Abwassers dient. Diese Probenentnahmeleitung 8 ist im Maschinenhaus 7 vor der Förderpumpe 6 aus der Förderleitung 8 herausgeführt und an eine Dosierpumpe 9 angeschlossen. Das andere Mündungsende der Probenentnahmeleitung 8 endet im oberen Bereich des Sedimentationsgefäßes 4, in dem sich nach einem Sedimentationsvorgang die Klarphase befindet. Bei der Probenentnahme arbeitet die Dosierpumpe 9 im Saugbetrieb und entnimmt eine bestimmte Menge des Abwassers aus der Klarphase im Sedimentationsgefäß 4 als Abwasserprobe.

Die Abwasserprobe wird dann einer photometrischen Auswertungseinheit 10 zugeführt. Die Auswertungseinheit 10 versetzt zunächst eine bestimmte Menge der Abwasserprobe mit einer bestimmten Menge schwefelsaurer Molybdat-Vanadat-Lösung, die mit dem Abwasser zu der gelben Phosphor-Vanadomolybdänsäure (P-V-Mo-Säure) reagiert, wobei die Intensität der Gelbfärbung und damit die Lichtabsorption von der Phosphat-Konzentration im Abwasser abhängt.

Anschließend durchleuchtet die Auswertungseinheit 10 die Abwasserprobe und mißt mittels einer Photozelle die Intensität des von der Abwasserprobe durchgelassenen Lichts. Daraus wird dann die Lichtabsorption und die Phosphat-Konzentration berechnet. Zur Berechnung und zur Anzeige der Phosphat-Konzentration sowie zur Steuerung der Anlage ist ein Steuerrechner 11 vorgesehen.

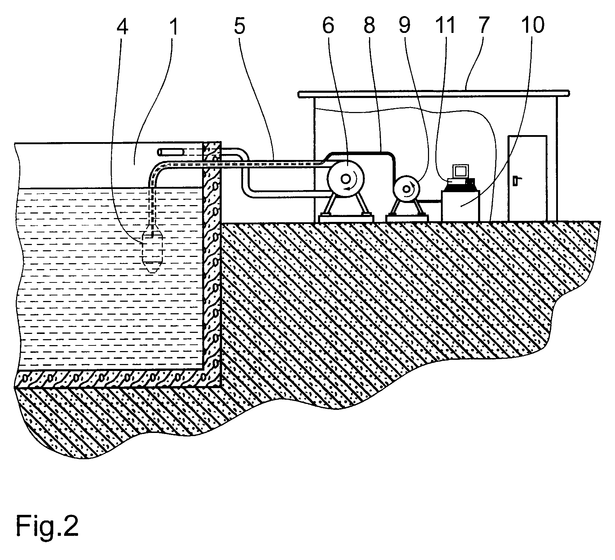

Durch die in Figur 2 gezeigte Querschnittsdarstellung der Anordnung aus Figur 1 wird deren Aufbau weiter verdeutlicht. In dieser Darstellung ist gut zu erkennen, daß das Abwasserbecken 1 ca. 1,2 m tief ins Erdreich eingelassen ist. Das Sedimentationsgefäß 4 ist dabei unterhalb des Wasserspiegels im Abwasserbecken 1 angeordnet. Die Sedimentation des Abwassers findet also innerhalb des Abwasserbeckens 1 statt.

Der Sedimentationsvorgang wird dabei durch die Strömungen im Abwasserbecken 1 nur unwesentlich behindert, da das Sedimentationsgefäß 4 bis auf die Auffüllöffnung geschlossen ist und die Einfüllöffnung aufgrund ihres relativ kleinen Querschnitts einer von außen in das Sedimentationsgefäß 4 eindringenden Strömung einen relativ großen Strömungswiderstand entgegensetzt.

Das Abwasser wird im Abwasserbecken 1 von dem an dem Ausleger befestigten Rechen umgerührt. Die Hauptströmungsrichtung im Abwasserbecken 1 verläuft deshalb in horizontaler Richtung. Da die Auffüllöffnung an der Unterseite des Sedimentationsgefäßes 4 angeordnet ist und somit nicht in der Hauptströmungsrichtung liegt, wird das Innere des Sedimentationsgefäßes 4 deshalb nur unwesentlich beeinflußt.

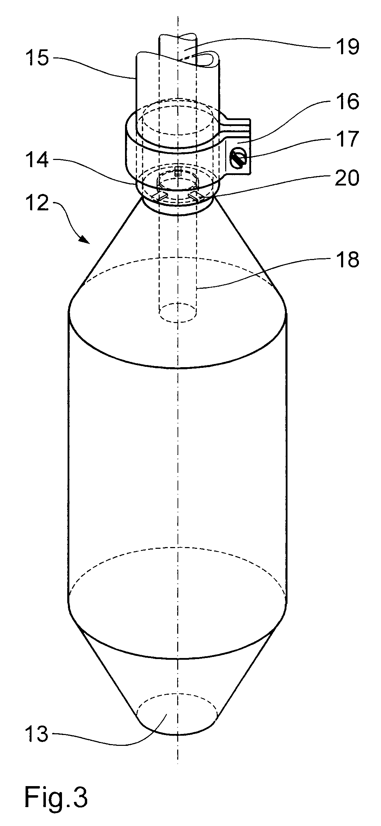

Aus Figur 3 ist der Aufbau eines erfindungsgemäßen Sedimentationsgefäßes 12 ersichtlich. Das Sedimentationsgefäß 12 ist als rotationssymmetrischer dünnwandiger Hohlkörper ausgeführt und besteht im wesentlichen aus Stahl.

In der Mitte des Sedimentationsgefäßes 12 befindet sich ein zylindrisches Mittelteil, das einen Durchmesser von im wesentlichen 20 cm und eine Höhe von im wesentlichen 15 cm aufweist und während des Sedimentationsvorgangs vollständig mit Abwasser gefüllt ist.

An das Mittelteil schließt sich ein Unterteil an, das sich nach unten hin verjüngt und in einer Öffnung 13 mit einem Durchmesser von ca. 1 cm ausläuft. Diese Öffnung 13 dient zum Befüllen des Sedimentationsgefäßes 12 mit Abwasser.

An das Mittelteil schließt an dessen Oberseite ein konisches Oberteil an, das sich nach oben hin bis zu einem Durchmesser von im wesentlichen 3,81 cm (1½") verjüngt. An dieses Oberteil schließt sich dann ein hohlzylindrischer Flansch 14 an, der zur Montage der Förderleitung 15 dient und eine Länge von ca. 4 cm aufweist.

Zur Montage der Förderleitung 15 wird diese axial auf den Flansch 14 aufgeschoben und anschließend mit einer Rohrschelle 16 auf dem Flansch fixiert. Die Rohrschelle 16 umschließt die Förderleitung 15 im Bereich des Flansches 14 und preßt die Förderleitung 15 von außen radial auf den Flansch 14. Zwischen Flansch 14 und Förderleitung 15 entsteht deshalb eine reibschlüssige Verbindung.

Da das Sedimentationsgefäß 12 im Abwasser angeordnet wird, kann während des Betriebs aufgrund der Kapillarwirkung Abwasser in den Zwischenraum zwischen Flansch 14 und Förderleitung 15 eindringen und die Reibungskraft herabsetzen. Damit sich der Förderschlauch 15 auch im Abwasser nicht vom Sedimentationsgefäß 12 löst, ist deshalb an der Außenwand des Flanschs 14 eine umlaufende Riffelung angebracht, die sich beim Anziehen der Spannschraube 17 der Rohrschelle 16 in die Innenwand der Förderleitung 15 hineindrückt und einen festen Sitz der Förderleitung 15 sicherstellt.

In dem Sedimentationsgefäß 12 ist ein Ansatzstück 18 zur Montage der Probenentnahmeleitung 19 angebracht. Das Ansatzstück 18 ist ein hohlzylindrisches dünnwandiges Rohr aus Stahl mit einer Länge von ca. 8 cm, das mittels mehrerer Streben 20 zentrisch im Flansch 14 fixiert ist und nach unten ca. 4 cm in das Sedimentationsgefäß 12 hineinragt. Das unten liegende Mündungsende des Ansatzstücks 18 befindet sich also im oberen Bereich des Sedimentationsgefäßes 12. Dies ist wichtig, damit bei der Entnahme einer Abwasserprobe durch die Probenentnahmeleitung 18 das Abwasser aus der Klarphase im Sedimentationsgefäß 12 entnommen wird. Die Probenentnahmeleitung 19 wird zur Montage einfach auf das obere Mündungsende des Ansatzstückes 18 aufgeschoben. Damit die Probenentnahmeleitung 19 nicht vom Ansatzstück 18 abrutscht, weist dieses an seiner Außenwand eine umlaufende Riffelung auf, die sich beim Aufschieben der Probenentnahmeleitung 19 in deren Innenwand hineindrückt und so für einen sicheren Sitz der Probenentnahmeleitung 19 sorgt.

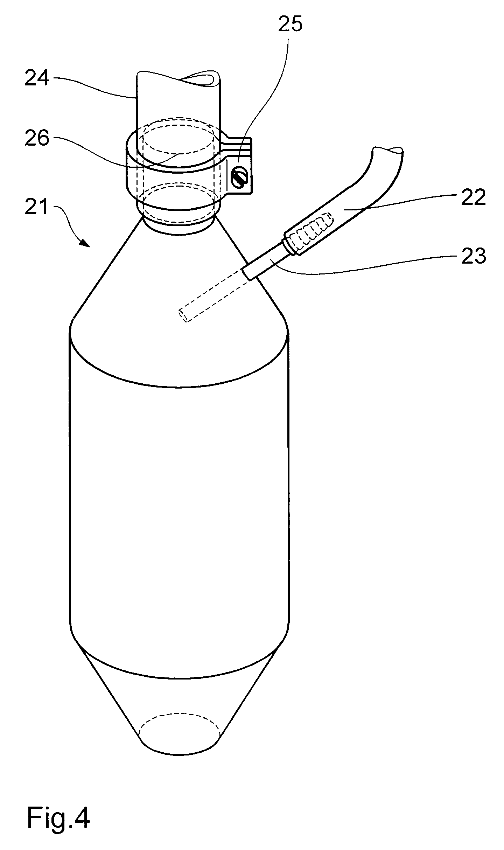

Die in Figur 4 dargestellte Ausführungsform eines erfindungsgemäßen Sedimentationsgefäßes 21 unterscheidet sich von dem in Figur 3 dargestellten Sedimentationsgefäß im wesentlichen durch den Anschluß der Probenentnahmeleitung 22 an das Sedimenationsgefäß 21, während die Form des Sedimentationsgefäßes 21 mit dem in Figur 3 dargestellten Sedimentationsgefäß im wesentlichen übereinstimmt.

Bei dieser Ausführungsform ist zur Montage der Probenentnahmeleitung 22 ein Ansatzstück 23 vorgesehen, das das konische Oberteil des Sedimentationsgefäßes 21 an seiner Mantelfläche durchstößt.

Das Ansatzstück 23 ist als hohlzylindrisches Stahlrohr mit einer Länge von ca. 12 cm und einem Außendurchmesser von ca. 1,27 cm (½") ausgeführt und so angeordnet, daß das innenliegende Mündungsende im oberen Bereich des Sedimentationsgefäßes 21 liegt. Beim Ansaugen einer Abwasserprobe wird deshalb schwebstoffarmes Abwasser aus der Klarphase im Sedimentationsgefäß 21 angesaugt.

Die Montage der Probenentnahmeleitung 22 erfolgt durch Aufschieben der Probenentnahmeleitung 22 auf die außerhalb des Sedimentationsgefäßes 21 liegende Mündungsöffnung des Ansatzstücks 23. Damit die Probenentnahmeleitung 22 nicht von dem Ansatzstück 23 abrutscht, ist an der Außenwand des Ansatzstücks 23 im Bereich der außenliegenden Mündungsöffnung eine umlaufende Riffelung angeformt, die sich beim Aufschieben der Probenentnahmeleitung 22 in deren Innenwand hineindrückt und damit einen festen Sitz der Probenentnahmeleitung 22 auf dem Ansatzstück 23 sicherstellt. Zusätzlich kann die Probenentnahmeleitung 22 durch eine Rohrschelle auf dem Ansatzstück 23 gesichert werden. Hierzu wird die Rohrschelle im Bereich der Mündungsöffnung des Ansatzstücks 23 über die Probenentnahmeleitung 22 geschoben und preßt dann die Probenentnahmeleitung 22 radial auf das Ansatzstück 23, so daß eine reibschlüssige Verbindung von Ansatzstück 23 und Probenentnahmeleitung entsteht.

Der Anschluß der Förderleitung 24 an das Sedimentationsgefäß 21 erfolgt wie bei dem in Figur 3 dargestellten Sedimentationsgefäß durch Aufschieben der Förderleitung 24 auf einen am oberen Ende des Sedimentationsgefäßes 21 angeformten hohlzylindrischen Flansch 26 und anschließendes Sichern der Verbindung durch eine Rohrschelle 25, die im Bereich des Flansches 26 über die Förderleitung 24 geschoben wird und diese radial auf den Flansch 26 preßt. Hierdurch entsteht eine reibschlüssige Verbindung von Flansch 26 und Förderleitung 24.9

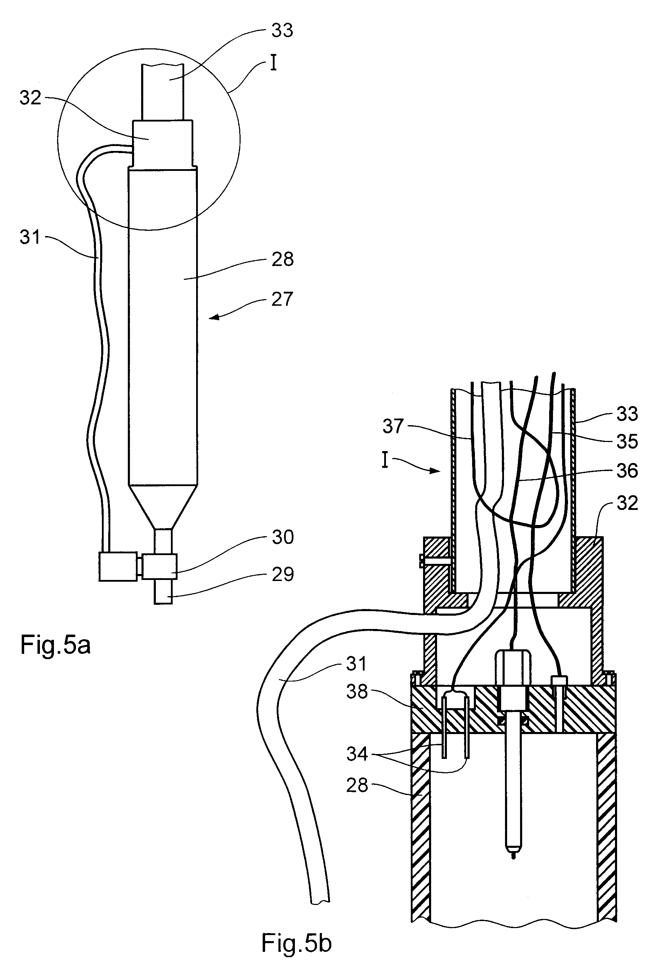

Das in den Figuren 5a und 5b als weiteres Ausführungsbeispiel gezeigte Sedimentationsgefäß 27 besteht im wesentlichen aus einem Polyethylen-Rohr 28 zylindrischen Querschnitts mit einem Durchmesser von 75 mm, das sich an seinem unteren Ende konisch verjüngt und in einem kurzen Endstück 29 mit einem Durchmesser von ca. 15 mm ausläuft. In diesem Endstück 29 ist ein Magnetventil 30 angeordnet, das ein hermetisches Abdichten des Sedimentationsgefäßes 27 an der Unterseite ermöglicht.

An seinem oberen Ende ist das PE-Rohr 28 durch eine Deckplatte 38 abgeschlossen, auf deren Oberseite ein Anschlußstück 32 festgeschraubt ist, das die Verbindung des PE-Rohrs 28 mit einem Aluminiumrohr 33 mit einem Durchmesser von 40 mm ermöglicht.

In der Deckplatte 38 befinden sich mehrere Durchbrüche, durch die ein Füllstandssensor 34, eine Probenleitung 36 sowie eine Druck-/Saugleitung 35 hindurchgeführt sind. Die Probenleitung 36 dient hierbei zur Entnahme einer Abwasserprobe aus der sich in dem Sedimentationsgefäß 27 nach einem Sedimentationszeitraum bildenden Klarphase, während die Druck-/Saugleitung 35 lediglich zur Befüllung und Entleerung des Sedimentationsgefäßes mit einer Abwasserprobe dient.

Probenleitung 36, Druck-/Saugleitung 35 sowie die Zuleitung für den Füllstandssensor 34 sind durch das Aluminiumrohr 33 aus dem Abwasserbecken herausgeführt und mit einer außerhalb des Abwasserbeckens angeordneten Steuereinheit verbunden. Darüber hinaus nimmt das Aluminiumrohr 33 noch die Steuerleitung 31 für das Magnetventil 30 sowie eine kabelförmige, elektrisch betriebene Rohrbegleitheizung 37 auf, die aufgrund der ohmschen Verluste eine Heizleistung von 8-10 W/m in das Aluminiumrohr einkoppelt und so ein Einfrieren der Probenleitung 36 und der Druck-/Saugleitung 35 verhindert.

Die Anordnung von Füllstandssensor 34, Probenleitung 36, Druck-/Saugleitung 35 sowie Rohrbegleitheizung 37 wird aus der in Figur 5b gezeigten Querschnittsdarstellung des Abschnitts I aus Figur 5a ersichtlich.

Zur Entleerung des Sedimentationsgefäßes 27 wird zunächst das Magnetventil 30 geöffnet und mittels einer an die Druck-/Saugleitung 35 angeschlossenen, außerhalb des Abwasserbeckens angeordneten Pumpe Luft in das Sedimentationsgefäß 27 gepumpt, so daß das in dem Sedimentationsgefäß 27 befindliche Abwasser nach unten herausgedrückt wird.

Um das Sedimentationsgefäß 27 mit Abwasser bzw. Belebtschlamm zu füllen, wird dann anschließend bei geöffnetem Magnetventil 30 durch die Druck-/Saugleitung 35 Luft aus dem Sedimentationsgefäß 27 abgesaugt, so daß infolge des in dem Sedimentationsgefäß 27 sich ausbildenden Unterdrucks Abwasser an der Unterseite durch das geöffnete Magnetventil 30 angesaugt wird. Das Sedimentationsgefäß 27 wird auf diese Weise so weit aufgefüllt, bis der Flüssigkeitsspiegel den Füllstandssensor 34 erreicht.

Anschließend wird das Magnetventil 30 geschlossen, so daß sich die in dem Sedimentationsgefäß 27 befindliche Abwasserprobe beruhigen kann. Während eines Sedimentationszeitraums von ca. 5-10 min. setzen sich dann die in der Abwasserprobe enthaltenen Schlammflocken am Boden des Sedimentationsgefäßes 27 ab, so daß im oberen Bereich des Sedimentationsgefäßes 27 eine feststoffarme Klarphase entsteht.

Während der anschließenden Förderphase wird dann bei geschlossenem Magnetventil 30 durch die Druck-/Saugleitung 35 Luft in das Sedimentationsgefäß 27 gepumpt, so daß Abwasser aus der Klarphase in die Probenleitung 36 hineingepreßt wird und so die außerhalb des Abwasserbeckens angeordnete Steuereinheit erreicht. Im Gegensatz zu einer Saugpumpe, deren theoretisch maximal erreichbare Förderhöhe auf ca. 10 m begrenzt ist, lassen sich auf diese Weise vorteilhaft auch größere Förderhöhen realisieren.

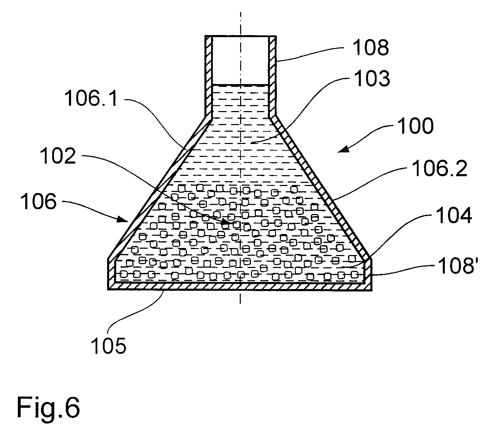

Das in Figur 6 dargestellte Sedimentationsgefäß 100 weist einen geschlossenen Boden 105 auf und enthält Schlamm- und/oder Feststoffpartikel aufweisendes Abwasser 102. Das Abwasser 102 wird nach Ablauf der Sedimentationszeit in eine im wesentlichen partikelfreie Klarphase 103 und in eine in überwiegendem Maße Schlamm- und/oder Feststoffpartikel enthaltende Phase 104 separiert.

Das Sedimentationsgefäß 100 weist drei unterschiedlich ausgebildete Zonen 106, 108, 108' auf. Zwischen einer oberen, zylindrisch ausgebildeten Zone 108 des Sedimentationsgefäßes 100 und einer den Boden 105 aufweisenden, zylindrischen Zone 108' ist eine sich in Richtung des Gefäßbodens 105 erweiternde Zone 106 vorgesehen. Diese Zone ist kegelstumpfförmig ausgebildet, wobei sich die Wandungsabschnitte 106.1 und 106.2 unter einem gleich großen, spitzen Winkel gegeneinander geneigt erstrecken.

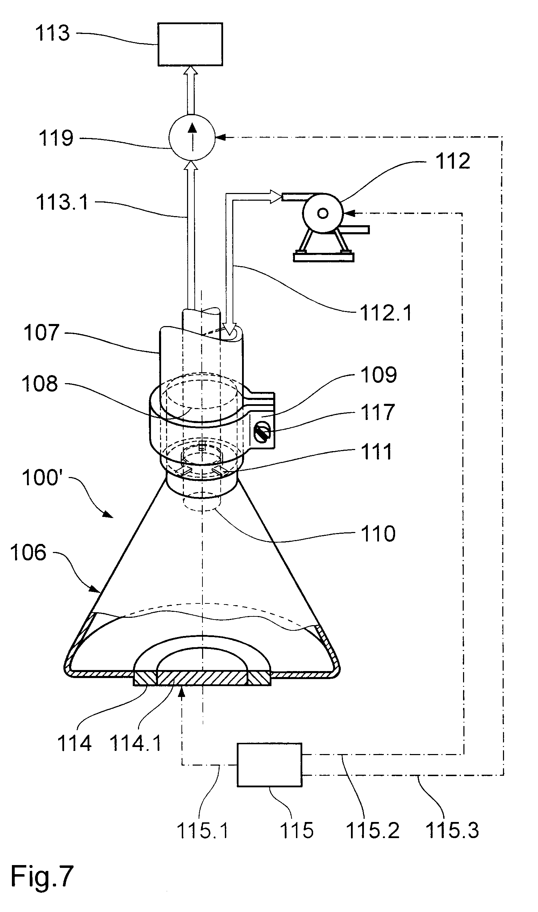

Figur 7 zeigt ein ungefülltes Sedimentationsgefäß 100' mit einer Zone 106 in Form eines sich in Richtung des Gefäßbodens erweiternden, geraden Kegelstumpfes. Der Neigungswinkel der Wandung des Kegelstumpfes in Bezug auf die Senkrechte beträgt im wesentlichen 45°. Die Grundfläche des Kegelstumpfes 106 stimmt mit dem Boden 105 des Sedimentationsgefäßes 100' überein. An der oberen, zylindrisch ausgebildeten, Zone 108 ist eine erste Leitung 110 zur Entnahme einer Probe aus der Klarphase (vergleiche die Position 103 in Figur 6) angeordnet. Er bildet zusammen mit der auf gleicher Achse liegenden zweiten Leitung 107 für die das zu prüfende Abwasservolumen in bzw. aus dem Sedimentationsgefäß 100' bewegende Förderpumpe 112 eine Rohr-in-Rohr-Anordnung. Die Entnahmeleitung 110 ist hierbei innerhalb des Rohrabschnitts 108 des Sedimentationsgefäßes 100' durch Abstandhalter 111 arretiert, wobei die zweite, auf die Zone 108 aufgeschobene Leitung 107 mittels einer Schelle 109 in ihrer Position fixiert ist.

Am Boden 105 des Sedimentationsgefäßes 100' ist eine Verschlußeinrichtung 114 vorgesehen, deren Durchlaß durch ein Magnetventil 114.1 steuerbar ausgebildet ist. Über den Gefäßboden 105 wird das zu untersuchende Abwasservolumen (vergleiche die Position 102 in Figur 6) in das Sedimentationsgefäß 100' eingebracht. Nach erfolgter Probenentnahme aus der sich bildenden Klarphase wird das gesamte Abwasservolumen im wesentlichen vollständig wieder aus dem Sedimentationsgefäß 100' entfernt.

Der für Einbringen und Entfernen des Flüssigkeit erforderliche Druck wird durch eine Förderpumpe 112 mit Drehrichtungsumkehr, welche über die Leitung 107 mit dem Sedimentationsgefäß 100' verbunden ist, erzeugt. Die aus der Klarphase entnommene Probe wird über die Leitung 110 durch die Pumpe 119 einer optischen Meß- und Auswerteeinrichtung 101, 113 zugeführt.

Die Bewegungsrichtung der entsprechenden Flüssigkeitsströme ist durch die Pfeile 112.1 und 113.1 gekennzeichnet.

Zur zeitlichen Koordinierung des Betriebes der Förderpumpen 112 bzw. 119 und des Magnetventils 114.1 der Verschlußeinrichtung 114 ist eine Steuerung 115 vorgesehen, welche über Steuerleitungen 115.1, 115.3 und 115.3 mit der Förderpumpe 112 für den Transport der Abwassermenge, die Förderpumpe 119 für den Transport der aus der Klarphase entnommenen Probe und der optischen Meß- und Auswerteeinrichtung 113 verbunden ist.

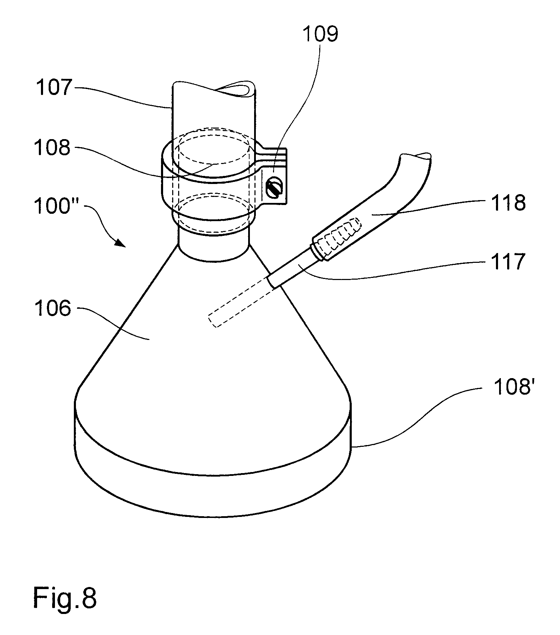

Das in Figur 8 dargestellte Sedimentationsgefäß 100'' weist einen zylindrisch ausgebildeten oberen Zone 108 und einem unteren Zone 108' größeren Durchmessers auf, welcher den Gefäßboden trägt. Zur Verbindung des unteren und des oberen Zones 108, 108' ist eine kegelstumpfförmige Zone 106 vorgesehen, welcher sich in Richtung des Gefäßbodens erweitert.

Die Leitung 118 zur Entnahme der Probe aus der Klarphase ist über einen in die kegelstumpförmige Zone 106 eingesetzten Stutzen 117 mit dem Sedimentationsgefäß 100'' verbunden. Die Leitung 107 zum Anschluß einer Förderpumpe zum Transport der Abwassermenge in das oder aus dem Sedimentationsgefäß 100'' ist mittels einer Schelle 109 an dem oberen Zone 108 befestigt.

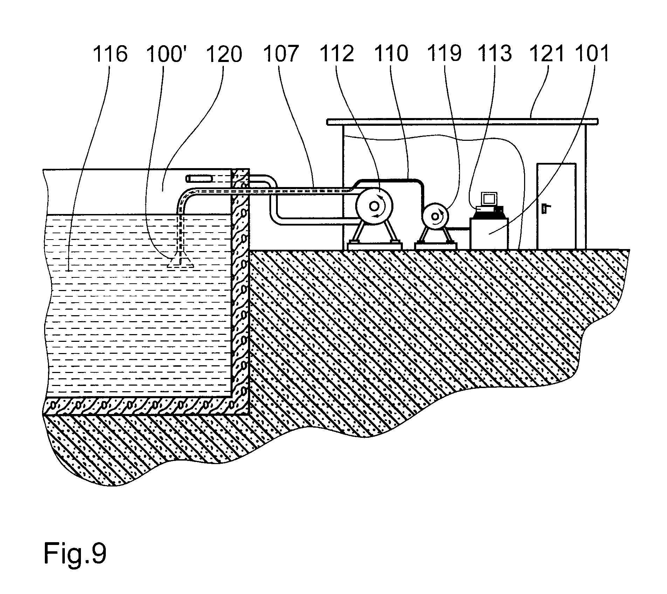

In dem in Figur 9 dargestellten Teilschnitt einer Kläranlage mit einem Abwasserbecken 120 ist ein Sedimentationsgefäß 100' gezeigt, welches vollständig von zu untersuchendem Abwasser 116 umgeben ist.

Über die Leitung 107 wird in Verbindung mit der in der Meßstation 121 befindlichen Förderpumpe 112 eine zu untersuchende Abwassermenge in das Sedimentationsgefäß 100' gesaugt. Nach erfolgter Sedimentation, welche aufgrund des durch einen Boden geschlossenen Sedimentationsgefäßes 100' ungestört durch Strömungen innerhalb des Abwasserbeckens 120 stattfindet, kann über die Leitung 110 mittels einer im Saugbetrieb arbeitenden Dosierpumpe 119 eine Probe aus der in dem Sedimentationsgefäß 100' entstandenen Klarphase entnommen und in die optischen Meß- und Auswerteeinrichtung 113 eines Meßplatzes eingeleitet werden.

Zur Bestimmung des Phosphatgehaltes des Abwassers 116 wird zunächst einer bestimmte Menge die der Klarphase entnommene Probe in der optischen Meß- und Auswerteeinheit 113 mit einer bestimmten Menge schwefelsaurer Molybdat-Vanadat-Lösung versetzt, welche mit dem Abwasser in Abhängigkeit von dessen Phosphatgehalt zu einer gelben Phosphor-Vanadomolybdänsäure (P-V-Mo-Säure) reagiert. Die Intensität der Gelbfärbung und damit auch das Maß der Lichtabsorption ist der Phosphatkonzenttration im Abwasser direkt proportional.

Anschließend durchleuchtet die optische Meß- und Auswerteeinheit 113 die Probe und mißt mittels einer Photozelle die Intensität des die Probe passierenden Lichts. Daraus wird die Lichtabsorption und die Phosphatkonzentration berechnet.

Zur Berechnung und Anzeige der meßtechnisch ermittelten Phosphatkonzentration sowie zur Steuerung der gesamten Anlage ist ein Steuerrechner 101 vorgesehen, welcher auch die nicht dargestellte Steuerung (vergleiche die Position 115 in Figur 7) für einen zeitlich synchronen Betrieb der Förderpumpe 112 und der Verschlußeinrichtung am Boden des Sedimentationsgefäßes 100' bzw. zur zeitgerechten Aktivierung der Dosierpumpe 119 aufweist.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei grundsätzlich anders gearteten Ausführungen Gebrauch macht.

## Patentansprüche

1. Vorrichtung zur Entnahme einer feststoffarmen Abwasserprobe zur Bestimmung gelöster Wasserinhaltsstoffe, insbesondere zur Messung der Phosphat-Konzentration im Abwasserbecken (1, 120) einer Kläranlage, mit
einem vibrationsarm gelagerten Sedimentationsgefäß (4, 12, 21, 100, 100', 100'') zur Aufnahme von Abwasser und zur Trennung von Schwebstoffen (104) und Klarphase (103) aus dem Abwasser mit einer Öffnung (13, 114) zum Einbringen des Abwassers (102, 116),
einer im oberen Bereich des Sedimentationsgefäßes (4, 12, 21, 100, 100', 100'') mündenden ersten Leitung (8, 19, 22, 110, 118) zur Entnahme einer Abwasserprobe aus der Klarphase (103),
**dadurch gekennzeichnet**,
daß das Sedimentationsgefäß (4, 12, 21, 100, 100', 100'') derart gehalten ist, daß sich mindestens seine Öffnung (13, 114) unterhalb des Flüssigkeitsspiegels des Abwasserbeckens befindet, und
daß die Öffnung des Sedimentationsgefäßes (4, 12, 21, 100, 100', 100'') zur Beruhigung des aufgenommenen Abwassers während des Sedimentationszeitraums durch ein Ventil (30, 114.1) verschließbar ist oder einen derart großen Strömungswiderstand aufweist, daß die Geschwindigkeit von Teilchen innerhalb des Sedimentationsgefäßes (4, 12, 21, 100, 100', 100'') derart verringert ist, daß sie sich innerhalb des Gefäßes absetzen.

2. Vorrichtung nach einem der vorhergehenden Ansprüche, **dadurch gekennzeichnet**, daß die erste Leitung (8, 110) mit einer außerhalb des Abwasserbeckens angeordneten Saugpumpe (9, 119) zum Absaugen der Abwasserprobe verbunden ist.

3. Vorrichtung nach einem der vorhergehenden Ansprüche, **dadurch gekennzeichnet**, daß zum Einsaugen des Abwassers in das Sedimentationsgefäß (4, 12, 21, 100, 100', 100'') und/oder zum Hineinpressen des in dem Sedimentationsgefäß (4, 12, 21, 100, 100', 100'') befindlichen Abwassers in die erste Leitung (8, 110) eine in das Sedimentationsgefäß mündende zweite Leitung (5, 107) vorgesehen ist, wobei insbesondere die zweite Leitung (5, 107) mit einer außerhalb des Abwasserbeckens angeordneten Pumpe (6, 112) verbunden ist.

4. Vorrichtung nach einem der vorhergehenden Ansprüche, **dadurch gekennzeichnet**, daß die erste Leitung (8, 110) zumindest auf einem Teil ihrer Länge im Inneren der zweiten Leitung (5, 107) im wesentlichen koaxial zu dieser verläuft und/oder daß die erste Leitung (8, 110) und/oder die zweite Leitung (5, 107) zumindest auf einem Teil ihrer Länge eine Heizeinrichtung zur Verhinderung des Einfrierens aufweisen, wobei insbesondere die Heizeinrichtung ein elektrisch betriebenes, aus kaltleitendem Material bestehendes Heizelement (37) aufweist.

5. Vorrichtung nach einem der vorhergehenden Ansprüche, **dadurch gekennzeichnet**, daß in der Öffnung (13) ein Filter zur Grobfilterung des Abwassers angeordnet ist.

6. Vorrichtung nach einem der vorhergehenden Ansprüche, **dadurch gekennzeichnet**, daß das Ventil (30, 114.1) mit einer auf den Druck im Sedimentationsgefäß (4, 100') ansprechenden Steuervorrichtung angeordnet ist, wobei die Steuervorrichtung so ausbildet ist, daß das Ventil bei einem Unterdruck im Sedimentationsgefäß (4, 100') geöffnet, ansonsten jedoch geschlossen ist.

7. Vorrichtung nach einem der vorhergehenden Ansprüche, **dadurch gekennzeichnet**, daß vor der Öffnung (13) zu dieser beabstandet zur Verhinderung des Einströmens von Abwasser während des Sedimentationszeitraums eine Abdeckung angeordnet ist.

8. Vorrichtung nach einem der vorhergehenden Ansprüche, **dadurch gekennzeichnet**, daß das Sedimentationsgefäß (4, 100, 100', 100'') als rotationssymmetrischer Hohlkörper ausgeführt ist und/ um seine Symmetrieachse drehbar gelagert ist, daß ein Antrieb vorgesehen ist, um das Sedimentationsgefäß (4) in Drehung um seine Symmetrieachse zu versetzen, daß das erste Mündungsende der ersten Leitung (8) im wesentlichen zentrisch im Sedimentationsgefäß (4) angeordnet ist, wobei insbesondere die zweite Leitung (5, 107) eine im wesentlichen zylindrische Hohlwelle ist, die um ihre Längsachse drehbar gelagert ist.

9. Vorrichtung nach Anspruch 8, **dadurch gekennzeichnet**, daß das Sedimentationsgefäß (4) an seiner Innenwand zur Mitnahme des in seinem Inneren befindlichen Abwassers bei einer Drehung des Sedimentationsgefäßes (4) Erhebungen aufweist, die sich in axialer Richtung zumindest über einen Teil der Länge des Sedimentationsgefäßes (4) erstrecken.

10. Vorrichtung nach einem der vorhergehenden Ansprüche, **dadurch gekennzeichnet**, daß die erste Leitung (8) zur Zwischenlagerung der Abwasserprobe mit einem Auffanggefäß verbunden ist, wobei insbesondere zur Messung des Feststoffanteils der aus dem Sedimentationsgefäß (4, 100, 100', 100'') entnommenen Abwasserprobe an dem Auffanggefäß ein Photometer angeordnet ist und/oder das Auffanggefäß zum Zusetzen eines den Feststoffanteil herabsetzenden Fällungsmittels über einen Dispenser mit einem zur Aufnahme des Fällungsmittels dienenden Vorratsgefäß verbunden ist.

11. Vorrichtung, insbesondere nach einem der vorangehenden Ansprüche, **dadurch gekennzeichnet**, daß das Sedimentationsgefäß (100, 100', 100'') einen geschlossenen Gefäßboden (105) und eine im wesentlichen unterhalb der Entnahmeleitung vorgesehene Zone (106) aufweist, welche sich in Richtung des Gefäßbodens (105) erweitert.

12. Vorrichtung nach Anspruch 11, **dadurch gekennzeichnet**, daß die Zone (106) im wesentlichen kegelstumpfförmig ausgebildet ist.

13. Vorrichtung nach einem der Ansprüche 18 oder 19, **dadurch gekennzeichnet**, daß die Zone (106) als gerader Kegelstumpf ausgebildet ist und/oder eine Neigung von im wesentlichen 45° aufweist.

14. Vorichtung nach einem der Ansprüche 18 bis 20, **dadurch gekennzeichnet**, daß im oberen - nicht kegelstumpfförmigen - Gefäßbereich eine sich in axialer Richtung erstreckende erste Leitung (110) zur Entnahme der Probe aus der Klarphase (103) vorgesehen ist.

15. Vorrichtung nach Anspruch 23, **dadurch gekennzeichnet**, daß die Leitung (118) zur Entnahme der Probe aus der Klarphase (103) im kegelstumpfförmigen Gefäßbereich (106) angeordnet ist.
